# EUROPEAN PATENT APPLICATION

(11) **EP 4 074 835 A1**
(43) Date of publication of application: **19.10.2022**
(21) Application number: 21168526.8
(22) Date of filing: 15.04.2021
(51) Int. Cl.: C12N 15/86, A61P 31/12, A61P 35/00, C12N 15/11

(54) **H-1 PV EXPRESSING RNAI EFFECTORS**

(71) Applicant: Deutsches Krebsforschungszentrum - Stiftung des öffentlichen Rechts / Universität Heidelberg, 69120 Heidelberg (DE)
(72) Inventor: Li, Junwei, 69151 Heidelberg (DE); Leuchs, Barbara, 69121 Heidelberg (DE); Dahm, Michael W., 81667 Munich (DE)
(74) Representative: Schüssler, Andrea

(57) **Abstract**

The present invention relates to innovative protoparvoviruses (PV) expressing RNAi effectors, preferably shRNAs against the PD-L1 gene which display improved anticancer activity. These new viruses are based on the ΔH-1PVsilencer platform that consists of a protoparvovirus H-1PV featuring an in-frame deletion within the NS region (ΔH-1PV) and harbouring a shRNA expression cassette in which the expression of the shRNA is controlled by the H1 Polymerase III promoter. In this invention the inventors aimed to use the ΔH-1PVsilencer to silence the PD-L1 gene. PD1/PD-L1 negatively regulates T cell-mediated immune responses and serves as a mechanism for tumors to escape antigen-specific T cell immune responses. The present invention also provides cells or organisms comprising said parvovirus.

## Description

The present invention relates to innovative protoparvoviruses (PV) expressing RNAi effectors, preferably shRNAs. These new viruses are based on the ΔH-1PVsilencer platform that consists of a protoparvovirus H-1PV featuring an in-frame deletion within the NS region (ΔH-1PV) and harbouring a RNA expression cassette, preferably a shRNA cassette, in which the expression of the RNA is controlled by the Pol III H-1 promoter. The present invention also provides cells or organisms comprising said parvovirus.

### Background of the Invention

### RNAi interference, RNAi effectors and delivery

RNA interference (RNAi) was first recognized in *Caenorhabditis elegans* by Andrew Fire and Craig Mello, who later were awarded with the Nobel prize in 1998 for their discovery. The mechanism of RNAi is based on the sequence-specific degradation of host mRNAs by means of double-stranded RNAs complementary to the target sequence. RNAi is a naturally occurring cellular process that controls gene expression and therefore plays a pivotal role in many cellular processes, including development [1]. It is also a vital component of the immune response defending the cells against pathogens like viruses and transposons [2]. Soon after its discovery, RNAi technology was harnessed to address biological questions and treatment options for diseases owing its highly specificity and capacity to achieve potent knock-down of known genetic sequences [3]. Therapeutically, RNAi works via delivery of small RNA duplexes, including microRNA (miRNA) mimics, small interfering RNAs (siRNAs), short hairpin RNAs (shRNAs) and Dicer substrate RNAs (dsiRNAs) [4]. All the four classes of RNAi effectors are presently tested in a number of phase I-III clinical trials against various diseases including multiple types of cancer [5]. In particular, RNA interference (RNAi) is the process where the RNA silences the gene expression. It is initiated by the enzyme Dicer, which cleaves the double stranded RNA into smaller fragments. The passenger strand is further fragmented, and the guide strand is loaded on to RNA-induced Silencing Complex (RISC). The guide strand pairs with the target mRNA and Argonaute-2, a protein, which serves as the catalytic component of the RISC, cleaves the mRNA. Typically, a short-hairpin RNA (shRNA) is added through a plasmid. The shRNA undergoes nuclear processing and is exported in the cytoplasm where shRNA is processed into a short-interfering RNA (siRNA) along with the Dicer substrate RNA (dsiRNA). The shRNA binds to a specific sequence in the mRNA via RISC. This results in the degradation of mRNA.

The most common problem that RNAi encounter is the efficient delivery of the shRNA into the cells. There are several commercially available transfection reagents for effective delivery of shRNAs, however, certain types of cells are still difficult to transfect efficiently. Examples of viruses employed for the delivery and expression of shRNAs in cancer gene therapy are adenoviruses, adeno associated viruses, lentiviruses and retroviruses. Most of these viruses however are defective in replication and therefore the silencing effect is restricted to the primary infected cells. Due to safety concerns, the use of lentiviral vectors has been limited to the *in vitro* and cannot be taken further to the *in vivo* setting.

Examples of genes targeted by RNAi-mediated anticancer therapy are *KRAS, Polo-like kinase 1, Furin, Ephrin type-A receptor* and *c-myc.* Most of the clinical studies involve siRNAs conjugated to nanoparticles (e.g. lipid nanoparticles) which have superior stability in comparison to naked siRNAs. However despite significant improvements, a rapid decline of the siRNA is observed within the first 72 h post-injection with most of the siRNA taken up by hepatocytes (approximately 50% of the injected dose is found in the liver already after 30 min from injection). Thus, efficient delivery of siRNAs remains the major bottleneck in this field [5]. As most delivery systems are transient and intracellular concentration of the siRNAs is diluted during cell division, repeated administration of the siRNAs is often required.

Short hairpin RNAs (shRNAs) are another class of RNAi effectors [6]. shRNAs typically consist of two complementary (sense and antisense) 19-29 base pairs sequences separated by a short loop of 4-11 impaired nucleotides. Typically, expression is controlled by a RNA polymerase (Pol) III promoter (*e.g* U6, H1) or modified pol II promoters. After transcription of the shRNA, sense and antisense strand, connected by the loop, pair together forming the characteristic hairpin structure. This structure resembles the pre-miRNA that are naturally used by the cell to regulate gene expression and requires nuclear processing [3]. After the discovery of promoter-driven expression of shRNAs, the design of viral RNAi vectors became possible [7]. Examples of viruses employed for the delivery and expression of shRNAs in cancer gene therapy are adenoviruses, adeno-associated viruses, lentiviruses and retroviruses. Most of these viruses, however, are defective in replication and therefore the silencing effect is restricted to the primary infected cells.

### Oncolytic viruses and RNAi

Oncolytic viruses (OVs) are viruses which selectively replicate in and kill cancer cells and have the ability to spread throughout the tumour while sparing normal tissues. Their anticancer potential has been demonstrated at the preclinical level against a vast variety of tumour models and a number of OVs are presently under phase I-III clinical evaluation. In particular, Talimogene laherparepvec (T-Vec; Amgen), a modified herpes simplex virus (HSV) expressing the immunostimulatory cytokine granulocyte-macrophage colony-stimulating factor (GM-CSF), has been approved recently by FDA and EMA for the treatment of unresectable metastatic melanoma. However, the promising results obtained with OVs at the preclinical levels have not always been reproduced in clinic. Tumours are often highly heterogeneous in nature and it may be that certain tumours are moderately susceptible to virus-induced oncolysis. It is also possible that within a certain tumour a fraction of cells survives virus treatment leading to tumour re-growth. The application of RNAi technology in the context of OV-based therapy is therefore very attractive because it may represent a means to reinforce the efficacy of OVs by providing the virus an additional mode of action for killing those cancer cells that are poorly sensitive to its infection. In addition, OV-mediated delivery of RNAi effectors may overcome the general hurdle of RNAi technology how to achieve high-level expression of these molecules specifically in cancer cells, in particular after intravenous administration. Furthermore, OV can replicate and spread through the tumour thereby having the potential to amplify shRNA production and delivery. Arming OVs with RNAi effectors has proven to be a valid approach in the case of oncolytic adenoviruses (Ad) e.g. Ad expressing shRNAs targeting a number of tumour related genes including *VEGF, MYCN, SATB1, C-Met, Ki67, IL-8, hTERT and FAK* have superior anticancer activity than their parental viruses [8-15]. It was also shown that oncolytic HSV can be engineered to efficiently express RNAi effectors (shRNAs and artificial miRNAs) [16].

There exists already a first-generation ΔH-1PVsilencer platform (WO 2013/110464 A1) that consists of a ΔH-1PV virus genome that features an in-frame deletion encompassing nucleotides (nt) 2022 - 2135. The left (LP) and right palindromic sequence (RP) serve as self-priming origins of replication; P4 promoter regulates the expression of the NS gene, encoding the nonstructural proteins NS1 and NS2, and P38 promoter regulates the expression of the VP gene encoding the VP1 and VP2 viral proteins. The non-coding region (NCR) is downstream the VP region and a target-specific nucleic acid cassette is inserted into the non-coding region at nucleotide 4687 of the wild-type H-1PV genome. However, it has been found out that the target-specific nucleic acid cassette inserted at the *HpaI* site (positions 4686-4691) is not stably maintained and compromises the fitness of the virus.

In addition, a recombinant parvovirus, which contains the shRNA CDK9 expression cassette within the 3' end untranslated region of the virus genome at position 4570 is described in EP 3 327 124 A1. Although the virus is efficient in gene silencing and maintains its ability to replicate, the shRNA expression cassette is gradually lost during the propagation of the virus in the NB324K production cell line.

### Object of the Present Invention

As mentioned above, if shRNA is delivered by adenoviruses, adeno-associated viruses, lentiviruses and retroviruses, the viruses are often defective in replication and therefore the silencing effect is restricted to the primary infected cells.

Therefore, it is the object of the present invention to provide means for efficiently down-regulating the expression of cancer-related genes in a cell or organism in a more stable way.

According to the invention this is achieved by providing the subject matters defined in the claims. Preferred embodiments are mentioned in the dependent claims.

### Description of the present Invention

The present invention concerns a deletion variant of autonomous parvovirus H-1 expressing a RNAi effector, preferably shRNAs, targeting cancer-related genes or oncogenes, e.g. PD-L1.

H-1 PV have attracted high attention for their anti-cancer potential because they are non-pathogenic for humans and possess oncolytic and oncosuppressive properties. Pre-existing antiviral immunity is usually not a problem for these viruses as humans are normally not exposed to rodent parvovirus infection. The parvovirus genome consists of a single stranded DNA of approximately 5100 bases containing two promoters, P4 and P38 which regulate the expression of the non-structural (NS1 and NS2) and capsid (VP1 and VP2) proteins, respectively. Activation of the P4 promoter is a critical step in the PV life cycle. The activity of the P4 promoter is later modulated by its own gene product NS1, but its initial activation is completely dependent on host cellular factors, which are mainly expressed during the S phase of the cell cycle. This dependency, together with the fact that the virus is unable to stimulate quiescent cells to proliferate, contributes to the oncotropism of the virus, which replicates preferentially in proliferating, transformed or malignant cells. In addition, parvovirus cytotoxicity is also stimulated by cellular changes associated with neoplastic transformation. NS1 is the major viral toxic protein. H-1PV has been shown to activate several death pathways in cancer cells. Although the anticancer potential of PVs is supported by a large set of preclinical studies, efficacy can be expected to be a limiting factor in clinical applications. It is possible that some cancer cells are resistant to virus treatment, causing tumour relapse.

During the experiments resulting in the present invention it could be shown that the insertion of an shRNA expression cassette at a particular site of the parvovirus genome is compatible with parvovirus packaging capacity, does not interfere with viral replication and enhances the intrinsic cytotoxicity of the virus. The virus expresses high levels of shRNAs and is very efficient in gene silencing. The big advantage of H-1PV-silencer in comparison with replication defective vectors resides in its capacity to replicate and propagate in proliferating cells, e.g., cancer cells. Every infected/transduced cell theoretically could become a producer of novel viral particles. Progeny virions of through second rounds of infection could spread through the tumour and efficiently delivery and express therapeutic shRNAs. In this setting, the silencing signal could be amplified beyond the initial inoculum. Together, parvovirus-based vectors and shRNA technology compensate each other limitations: the natural oncotropism of parvovirus should specifically and effectively deliver to and mediate transduction of the shRNAs in proliferating cells, e.g., cancer cells.

### Brief description of the drawings

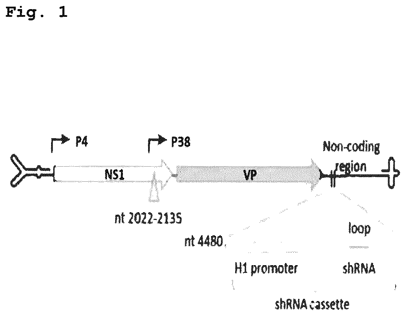

### Figure 1: Schematic representation of the ΔH-1PVRNAi genomic organization

The viral genome (top) is shown as a single line terminating in disparate hairpin telomeres which serve as self-priming origins of replication. The hairpins, drawn to represent their predicted structures, are scaled approximately 20× relative to the rest of the genome. The viral early P4 and late P38 promoters regulate the expression of the transcription units encoding the non-structural NS1 (arrowed light grey box) and capsid VP (arrowed dark grey box) proteins respectively. Transcriptional promoters are indicated by solid arrows. The grey triangle indicates the position of an in frame 114-nucleotide internal deletion. The non-coding region is downstream the VP region and it is supposed to be involved in the regulation of viral replication. The shRNA cassette (bottom), including H1 Pol III promoter and shRNA sequence, is inserted at nt 4480 within viral genome.

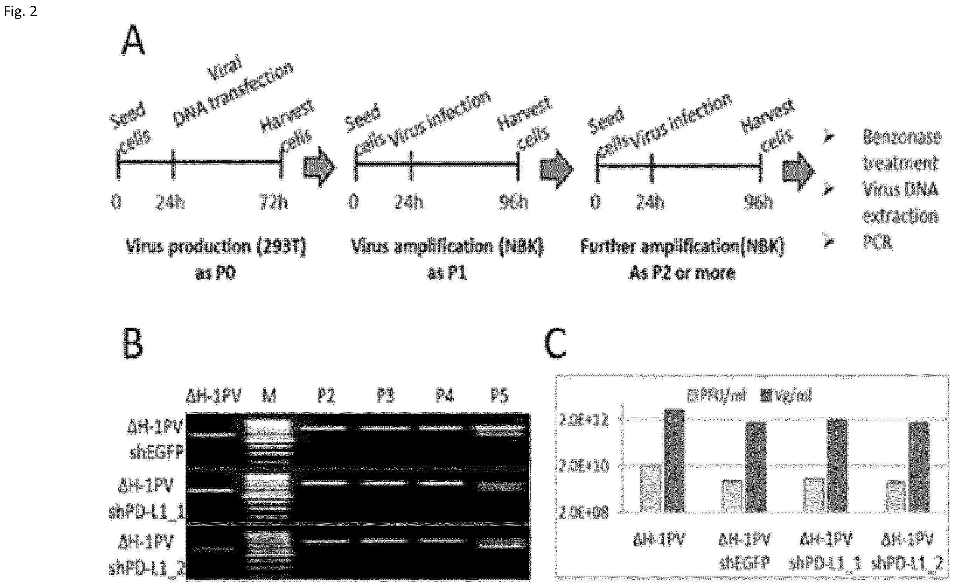

### Figure 2: Examination of stability of the shRNA expression cassette and production of ΔH-1PVshPD-L1

### A) Production scheme of ΔH-1PRNAi viruses in successive infection rounds

Virus production plan was adopted for checking the stability of the shRNA expression cassette via consecutive infection rounds as described in methods.

### B) Evaluation of stability of the shRNA expression cassette at nt4480

The viruses either ΔH-1PVshEGFP, ΔH-1PVshPD-L1_1 or ΔH-1PVshPD-L1_2, obtained from each passage P2, P3, P4 and P5 with procedures as shown in (A). The virus DNA was extracted and the genomic fragment including the shRNA cassette was amplified by PCR using primers flanking the cassette. The PCR product of ΔH-1PV was loaded as control.

### (C) Comparable progeny virus production between ΔH-1PV and ΔH-1PRNAi viruses

Titration of viruses were performed by plaque assay (light grey). Results are given from the representatives of the two independent experiments. Real-time PCR was conducted for quantification of the encapsidated viral genomes (dark grey). Results are presented with average of the two independent experiments.

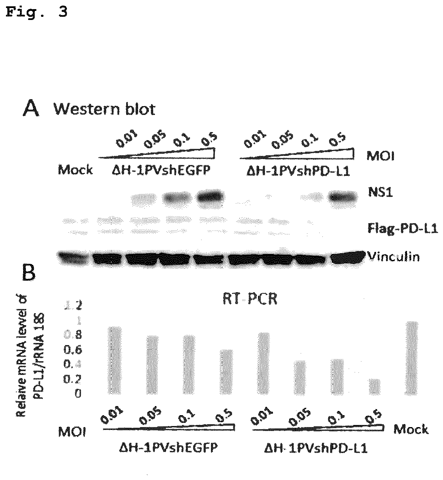

### Figure 3: ΔH-1PVshPD-L1 is efficient in gene silencing. (A) Western blot

U251 cells were infected with increased amount of MOI (PFU/cell) of the indicated viruses and grown for 72 h before to be lysed. Total cell extracts were subjected to SDS-PAGE followed by immunoblot analysis of the protein levels of Flag tagged PD-L1, NS1 and Vinculin (loading control) using specific antibodies.

### (B) qRT-PCR

U251 cells were infected as described in (A). Cells were harvested at 72 h post-infection and total RNA isolated and reverse transcribed. PD-L1 mRNA levels were quantified by qRT-PCR using specific primers. The expression levels of PD-L1 gene were normalized with housekeeping gene rRNA 18S. Values are expressed as relative to values obtained in mock-treated cells.

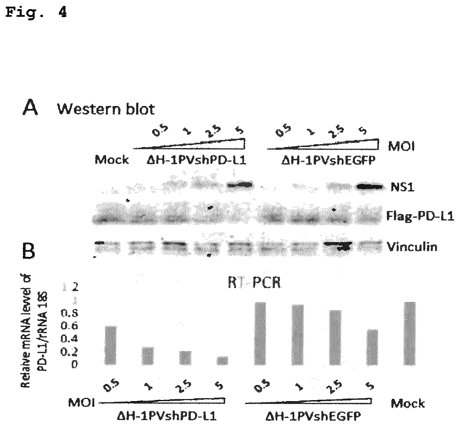

### Figure 4: ΔH-1PVshPD-L1 is potent in gene suppression.

### (A) Western blot

AsPC-1 cells were infected with increased amount of MOI (PFU/cell) of the indicated viruses and grown for 72 h before to be lysed. Western blot was performed as described in Fig.2 (A).

### (B) qRT-PCR

AsPC-1 cells were infected as the same as in (A). RT-PCR was carried out as described in Fig.2 (B).

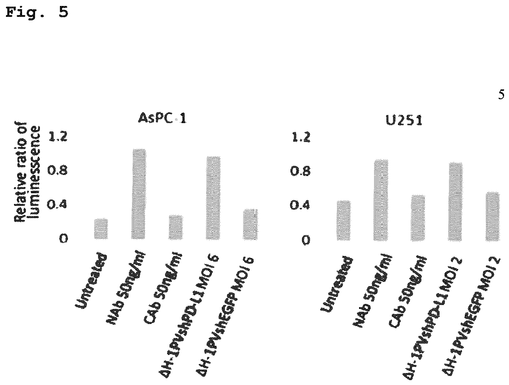

### Figure 5: Activation of the NFAT responsive luciferase reporter upon infection with ΔH-1PVshPD-L1

Either AsPC-1 (left panel) or U251 (right panel) cells were first transfected with TCR activator alone or TCR activator/PD-L1, and followed by infection with ΔH-1PVshPD-L1 as well as ΔH-1PVshEGFP as indicated MOI (PFU/cell). The infected cells were pre-incubated with either anti-PD-Ll neutralizing (NAb) or control antibodies (CAb) as illustrated amount (ng/ml) and followed by co-culture with Jurkat cells. The NFAT-luciferase reporter activity was measured by ONE-Step luciferase assay. Relative ratio of luminescence under different treatments was calculated as described in methods.

### Figure 6:

Parvovirus H-1, complete genome (SEQ ID NO:12)
GenBank: X01457.1

### Detailed Description of the Present Invention

Thus, the present invention provides a parvovirus based on a parvovirus H-1 deletion variant for down regulating the expression of a target gene in a cell characterized in that the parvovirus H-1 deletion variant contains a deletion encompassing the nucleotides 2022-2135, wherein the target specific nucleic acid is inserted in an untranslated region downstream of the H-1 parvovirus VP gene and is expressible under the control of a promoter or promoter region recognizable by an RNA polymerase in the cell, wherein said target specific nucleic acid is transcribable in an RNAi, and wherein said parvovirus is capable of replicating and propagating in the cell.

The parvovirus H-1 deletion variant (ΔH-1PV) features an in-frame deletion encompassing nucleotides (nt) 2022-2135 of wildtype parvovirus H-1 (Fig. 1). The left (LP) and right palindromic sequence (RP) serve as self-priming origins of replication; P4 promoter regulates the expression of the NS gene, encoding the nonstructural proteins NS1 and NS2, and P38 promoter regulates the expression of the VP gene encoding the VP1 and VP2 viral proteins. The non-coding region (NCR) is downstream the VP region and it is supposed to be involved in the regulation of virus genome replication and encapsidation.

The target specific nucleic acid is inserted into the viral genome in such a way that viral replication and cytotoxicity are not affected, i.e. downstream of the parvovirus VP gene encoding the capsid proteins of the parvovirus. Preferably, the target specific nucleic acid cassette is inserted at nucleotide 4480 of the ΔH-1PV genome. The inventors have conducted several tests to determine the most suitable insertion site. When the shRNA expression cassette was inserted at positions 4815 or 4465, the shRNA cassette was lost in passage 1 or 2. Inserting the shRNA expression cassette at positions 4598 or 4469 improves the stability of the cassette maintenance, i.e. it remains stably integrated into the viral genome in passage 3. This is even better than the insertion into the *Hpa*I restriction enzyme site used in the first generation ΔH-1PVsilencer platform (WO 2013/110464 A1). Best results were achieved when the shRNA expression cassette was inserted at position 4480. It is stably integrated into the viral genome in passage 4 with greater virus production as well as infectivity. The inventors could present a proof of concept that the novel cassette insert site at position 4480 is universal for any shRNAs.

In a preferred embodiment the target gene is PD-L1. PD-1 (programmed cell death 1) and PD-L1 (programmed cell death ligand 1) play a key role in negative regulation of T cell-mediated immune responses and serves as a mechanism for tumors to evade antigen-specific T cell immune responses. Thus, the blockade of PD-1 or PD-L1 is a suitable way for tumor treatment. Multiple additional immune-checkpoint receptors and ligands, some of which are selectively upregulated in various types of tumor cells, are prime targets for blockade, particularly in combination with approaches that enhance the activation of antitumor immune responses, such as vaccines. Presently, the blockade is achieved through the administration of anti-PDl or anti-PD-Ll antibodies. Anti-PD-Ll antibodies and methods of making the same are known in the art. Such antibodies to PD-L1 may be polyclonal or monoclonal, and/or recombinant, and/or humanized. Examples of antibodies to PD-L1 are disclosed in US Patent No. 8,217,149, US Application No. 13/511,538, US Application No. 13/478,511. Currently three antibodies against PD-L1, namely, atezolizumab, avelumab and durvalumab were approved by FDA. However, there are still unsolved problems associated with the administration of currently known checkpoint inhibitors:
(1) Adverse immune effects: the drawback of releasing the immune brake is the induction of toxicity in healthy tissues in the patients treated with immune checkpoint inhibitors (ICIs). The patients develop immune-related adverse events (irAEs), which are a unique spectrum of side effects of ICIs that resemble autoimmune responses. irAEs affect almost every organ of the body and are most commonly observed in the skin, gastrointestinal tract, lung, and endocrine, musculoskeletal and other systems. ICIs have significant clinical anticancer efficacy but often severe systemic toxicity. Thus, the incidence of irAEs of up to 50% has prevented their widespread and universal use.
(2) Mutation of the target protein PD-L1 results in treatment resistance
(3) for example, pancreatic ductal adenocarcinoma (PDAC) has demonstrated disappointing results in trials of single-agent immune checkpoint blockades. The possible reason for the failure may be due to the combination of immune escape mechanisms and low mutation burden in PDAC.

Therefore, it calls for new strategies for PD-L1 blockade as an anticancer therapeutic target gene since PD1/PD-L1 negatively regulates T cell-mediated immune responses and serves as a mechanism for tumors to escape antigen-specific T cell immune responses. In addition, it boosts cancer cell growth and promotes tumorgenesis.

In particular, the inventors generated the pΔH-1PVshPD-L1 and pΔH-1PVshEGFP infectious molecular clones by inserting shRNA expression cassettes within non-coding region of viral genome (Fig. 1). The shRNA sequences either against PD-L1 or for EGFP were proved with efficient gene specific silencing effects. To develop a novel H-1PV stably expressing shRNA cassette, the inventors have carried out several trials to evaluate the most suitable insertion site by examining the stability of shRNA expression cassettes into viral genome using successive infection rounds in permissive NBK cells as indicated in Fig. 2A. When the shRNA expression cassette was inserted at positions 4815 or 4465, the shRNA cassette was lost in passage 1 or 2. Inserting the shRNA expression cassette at positions 4598 or 4469 improves the stability of the cassette maintenance, i.e., it remains stably integrated into the viral genome in passage 3. This is even better than the insertion into the *Hpa*I restriction enzyme site used in the first generation ΔH-1PVshEGFP (WO 2013/110464 A1). Best results were achieved when the shRNA expression cassette was inserted at position 4480 (Fig. 2B). It is stably integrated into the viral genome in passage 4 with greater virus production as well as infectivity (Fig. 2B and C). This is the first time where evidence is provided that the optimal insertion site of the shRNA expression cassette is required for an adaptive compromise of parvovirus, suggesting that the virus has most favourable capacity to produce PVs expressing shRNA against the PD-L1 gene with good viral fitness. The virus production as well as infectivity were comparable in different constructs and not dependent on variations of shRNA sequences, i.e., either against PD-L1 or EGFP, indicating that the novel cassette insert site at position 4480 is universal for any shRNA sequences (Fig. 2C).

To select the most efficient shRNA sequences targeting PD-L1, the inventors developed the stable cell lines overexpressing Flag-tagged PD-L1 either in pancreatic ductal adenocarcinoma (PDAC) AsPC-1 or glioblastoma multiforme (GBM) U251 cells to evaluate the silencing efficiency upon infection with ΔH-1PVshPD-L1 bearing different shRNA sequences against PD-L1 gene. As a result, the inventors chose ΔH-1PVshPD-L1_1 with more potent in PD-L1 gene silencing according to the evaluation of knocking down efficacy for further analysis. Unless otherwise stated, the term 'ΔH-1PVshPD-L1_1' refers hereafter to ΔH-1PVshPD-L1. AsPC-1 or U251 cells overexpressing Flag-tagged PD-L1 were infected with increasing amounts (Fig. 3 and 4) of either ΔH-1PVshPD-L1 or ΔH-1PVshEGFP. After 72 h post-infection, cells were harvested for Western blot analysis on total cell lysates. The results indicated that there was a PD-L1 gene silencing effect with a viral titer-dependent manner upon infection by H-1PVshPD-L1 either in AsPc-1 or U251 cells, but not the control virus of ΔH-1PVshEGFP. In agreement with these results, a strong reduction of the PD-L1 mRNA levels was observed in ΔH-1PVshPD-L1 infected cells, confirming that the ΔH-1PVshPD-L1 has the ability to silence the PD-L1 gene.

The binding of programmed cell death protein 1 (PD-1), a receptor expressed on activated T cells, to its ligands, PD-L1 on most cancers, negatively regulates immune responses. The PD-1/PD-L1 interaction inhibits T cell activity and allows cancer cells to escape immune surveillance [35]. To explore the possibility that inhibition of PD-1/PD-L1 interaction could be achieved by silencing PD-L1 gene, the inventors tested if ΔH-1PVshPD-L1 can disrupt PD-1/PD-L1 interaction in a bioluminescent cell-based assay. Either AsPC-1 or U251 cells overexpressed PD-L1 and TCR activator were treated with anti-PD-L1 neutralizing antibodies as well as control antibodies, or infected with either ΔH-1PVshPD-L1 or ΔH-1PVshEGFP at a MOI (pfu/cell) of 12 (AsPC-1) and 2 (U251) as illustrated in Fig. 5. Luminescence corresponding to nuclear factor of activated T cells (NFAT) activity after co-cultured with PD-1/NFAT reporter Jurkat T cells was measured. As expected, the luminescence from treated with anti-PD-L1 neutralizing antibodies were 1,7 (U251) or 3,8 (AsPC-1)-fold higher than control antibodies. Interestingly, the luminescence from infected with ΔH-1PVshPD-L1 were 1,6 (U251) or 2,8 (AsPC-1)-fold higher than control virus ΔH-1PVshEGFP, suggesting that ΔH-1PVshPD-L1 could block and remove of PD1/PD-L1 interaction. The knock-down of PD-L1 gene was examined by western blot parallelly. These results indicated that ΔH-1PVshPD-L1 might play a role in promoting T cell activation via silencing PD-L1 gene.

This innovative virus represents the next generation of parvovirus which not only has the characteristics of the original H-1PV, but also has the competitive advantage with significant functions of gene silencing that other products do not show. The following results are a preclinical proof-of-concept that the ΔH-1PV pSilencer expressing shRNAs against the PD-L1 gene have a unique, superior anticancer profile through:
(1) H-1PV intrinsic ability of inducing tumor cell toxicity coupled with eliciting strong anticancer immune responses to switch the immunosuppressive tumor microenvironment (TME) of cancer cells from 'cold' to 'hot' status.
(2) Silencing of PD- L1 gene resulted in reversing the exhaustion of cytotoxic T lymphocytes, thus leading to the elimination of tumor cells via the re-induction of the 'natural' function of the T cell population.

The term "PD-L1 specific nucleic acid" as used herein refers to a nucleic acid comprising at least 15, 20, 25, 50, 100 or 200 consecutive nt having at least about 75%, particularly at least about 80%, more particularly at least about 85%, quite particularly about 90%, especially about 95% sequence identity with the complement of a transcribed nucleotide sequence of the PD-L1 target gene. The PD-L1 sequence is known and, for example, described in publications like Breton et al. [32], Wang et al. [36] and Jeffrey et al. [33]. Preferably, the following sequence was used for downregulation: 5'GATATTTGCTGTCTTTATA-3' (SEQ ID NO:1). With a RNA matching this sequence it was possible to target all known isoforms of PD-L1 (GenBank Accession No. NM_014143).

In the present invention the PD-L1 gene can be down regulated in an *in vivo* cell or an *in vitro* cell (ex vivo). The cell may be a primary cell or a cell that has been cultured for a period of time or the cells may be comprised of a cultured cell line. The cell may be a diseased cell, such a cancer cell or tumor or a cell infected by a virus. The cell may be a stem cell which gives rise to progenitor cells, more mature, and fully mature cells of all the hematopoietic cell lineages, a progenitor cell which gives rise to mature cells of all the hematopoietic cell lineages, a committed progenitor cell which gives rise to a specific hematopoietic lineage, a T lymphocyte progenitor cell, an immature T lymphocyte, a mature T lymphocyte, a myeloid progenitor cell, or a monocyte/macrophage cell. The cell may be a stem cell or embryonic stem cell that is omnipotent or totipotent. The cell maybe a nerve cell, neural cell, epithelial cell, muscle cell, cardiac cell, liver cell, kidney cell, stem cell, embryonic or foetal stem cell or fertilised egg cell.

Preferably, said parvovirus variant is formulated as a pharmaceutical composition, wherein the parvovirus is present in an effective dose and combined with a pharmaceutically acceptable carrier.

"Pharmaceutically acceptable" is meant to encompass any carrier which does not interfere with the effectiveness of the biological activity of the active ingredients and that is not toxic to the patient to whom it is administered. Examples of suitable pharmaceutical carriers are well known in the art and include phosphate buffered saline solutions, water, emulsions, such as oil/water emulsions, various types of wetting agents, sterile solutions etc.. Additional pharmaceutically compatible carriers can include gels, bioadsorbable matrix materials, implantation elements containing the parvovirus (therapeutic agent), or any other suitable vehicle, delivery or dispensing means or material(s). Such carriers can be formulated by conventional methods and can be administered to the subject at an effective dose.

An "effective dose" refers to amounts of the active ingredients that are sufficient to effect treatment. An "effective dose" may be determined using methods known to one skilled in the art (see for example, Fingl et al., The Pharmocological Basis of Therapeutics, Goodman and Gilman, eds. Macmillan Publishing Co., New York, pp. 1-46 ((1975)).

Administration of the parvovirus may be effected by different ways, e.g. by intravenous, intratumoral, intraperitoneal, subcutaneous, intramuscular, topical or intradermal administration. The route of administration, of course, depends on the kind of therapy. Preferred routes of administration are intravenous (i.v.), intratumoral or endobronchial administration. If infectious virus particles are used which have the capacity to penetrate through the blood-brain barrier, treatment could be performed or at least initiated by intravenous injection of, e.g., H1 virus.

The dosage regimen of the parvovirus is readily determinable within the skill of the art, by the attending physician based an patient data, observations and other clinical factors, including for example the patient's size, body surface area, age, sex, the particular modified parvovirus etc. to be administered, the time and route of administration, the type of mesenchymal tumor, general health of the patient, and other drug therapies to which the patient is being subjected.

As another specific administration technique, the parvovirus can be administered to the patient from a source implanted in the patient. For example, a catheter, e.g., of silicone or other biocompatible material, can be connected to a small subcutaneous reservoir (Rickham reservoir) installed in the patient, e.g., during tumor removal, or by a separate procedure, to permit the parvovirus to be injected locally at various times without further surgical intervention. The parvovirus can also be injected into a tumor by stereotactic surgical techniques or by neuronavigation targeting techniques.

Administration of the parvovirus can also be performed by continuous infusion of viral particles or fluids containing viral particles through implanted catheters at low flow rates using suitable pump systems, e.g., peristaltic infusion pumps or convection enhanced delivery (CED) pumps.

As yet another method of administration of the parvovirus is from an implanted device constructed and arranged to dispense the parvovirus to the desired tissue. For example, wafers can be employed that have been impregnated with the parvovirus, e.g., parvovirus H1, wherein the wafer is attached to the edges of the resection cavity at the conclusion of surgical tumor removal. Multiple wafers can be employed in such therapeutic intervention. Cells that actively produce the parvovirus H1 variant, can be injected into the tumor, or into the tumor cavity after tumor removal.

In a particularly preferred embodiment of the present invention, the target specific nucleic acid is inserted at position 4480 of the H-1PV genome. The underlying vector is pdB del H-1PV which is described in EP 2 397 542 A1.

In a further particularly preferred embodiment of the present invention, the promoter or promoter region recognizable by RNA polymerases is a RNA-polymerase II (Pol II) promoters such as for instance CMV, P38 and human ubiquitin C or RNA-polymerase III (Pol III) promoters such as U6, H1, 7SK and tRNA. An example of a particularly preferred RNA-polymerase III (Pol III) promoter is the RNA-polymerase III H1 promoter.

In a preferred embodiment of the present invention the PD-L1 specific nucleic acid is an shRNA. An shRNA is a small hairpin RNA or short hairpin RNA that is a sequence of RNA that makes a tight hairpin turn that can be used to silence gene expression via RNA interference. The shRNA hairpin structure is cleaved by the cellular machinery into siRNA, which is then bound to the RNA-induced silencing complex (RISC). This complex binds to and cleaves mRNAs which match the siRNA that is bound to it. However, the insertion of other RNAi triggering molecules such as microRNAs and/or antisense oligonucleotides is also possible.

In a further particularly preferred embodiment of the present invention, the PD-L1 specific nucleic acid, e.g., shRNA, has a length of at least 15 nucleotides. In a particular preferred embodiment the PD-L1 sequence matches the sequence 5'GATATTTGCTGTCTTTATA-3' (SEQ ID NO:1) of PD-L1 sequence as previously described [32]. The PD-L1 gene is alternatively spliced which results in multiple transcript variants. Out of the known four variants, one is non-coding and three codes for the different isoforms of PD-L1 protein are existing.

The present invention also relates to a rodent parvovirus as characterized above for use in treating cancer.

In a preferred embodiment, said parvovirus can be used for treating a tumour, in particular (but not exclusively) prostate cancer, pancreatic carcinoma, brain cancer (preferably gliomas), cervical carcinoma, lung cancer, head and neck cancer, breast cancer or colon cancer.

In a further preferred embodiment, said parvovirus can be used for the treatment of a tumour characterized in that the cells of said tumour are resistant to chemotherapy and/or radiotherapy.

Patients treatable by the parvovirus according to the invention include humans as well as non-human animals. Examples of the latter include, without limitation, animals such as cows, sheep, pigs, horses, dogs, and cats.

The present invention also provides a cell of an animal, fungus or protist comprising a parvovirus as hereinbefore described. In an embodiment, the cell is *in vitro.* The cell is preferably an animal cell, an isolated human cell, an *in vitro* human cell, a non-human vertebrate cell, a non-human mammalian cell, fish cell, cattle cell, goat cell, pig cell, sheep cell, rodent cell, hamster cell, mouse cell, rat cell, guinea pig cell, rabbit cell, non-human primate cell, nematode cell, shellfish cell, prawn cell, crab cell, lobster cell, insect cell, fruit fly cell, Coleapteran insect cell, Dipteran insect cell, Lepidopteran insect cell or Homeopteran insect cell.

Finally, the present invention also provides a transgenic, non-human animal, fungus or protist comprising a parvovirus as hereinbefore described. Transgenic animals can be produced by the injection of the parvovirus into the pronucleus of a fertilized oocyte, by transplantation of cells, preferably undifferentiated cells into a developing embryo to produce a chimeric embryo, transplantation of a nucleus from a recombinant cell into an enucleated embryo or activated oocyte and the like. Methods for the production of transgenic animals are well established in the art and, e.g., described in US patent 4,873, 191.

In summary, the new viruses are based on the ΔH-1PVsilencer platform that consists of a protoparvovirus H-1PV featuring an in frame-deletion within the NS region (ΔH-1PV) and harbouring an RNA expression cassette, preferably a shRNA expression cassette, in which the expression of the shRNA is controlled by the Pol III H1 promoter. The ΔH-1PVsilencer is effective in gene silencing while keeping its ability to replicate and be fully infectious. In the present invention the ΔH-1PVsilencer was used to silence the PD-L1 gene whose activity is known to cause an impaired functioning of the immune system. PD1/PD-L1 negatively regulates T cell-mediated immune responses and serves as a mechanism for tumors to escape antigen-specific T cell immune responses. Transfection of the plasmids in HEK293T cells generated fully infectious viral particles that can be further amplified via infection in NB324K cells following a classical parvovirus production protocol.

The below examples explain the invention in more detail.

### Example 1

### Plasmid construction and virus production

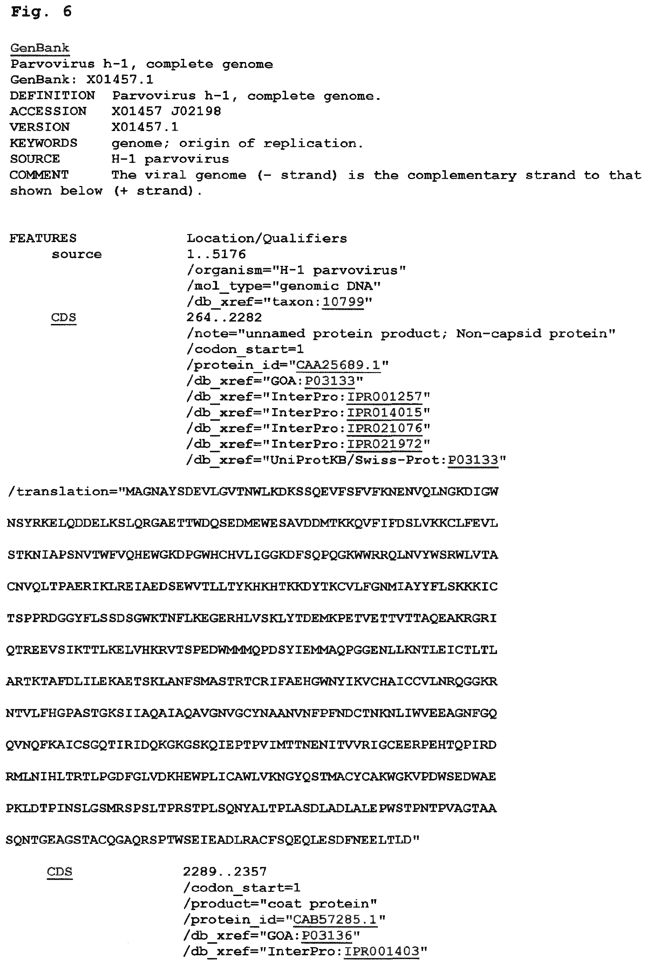

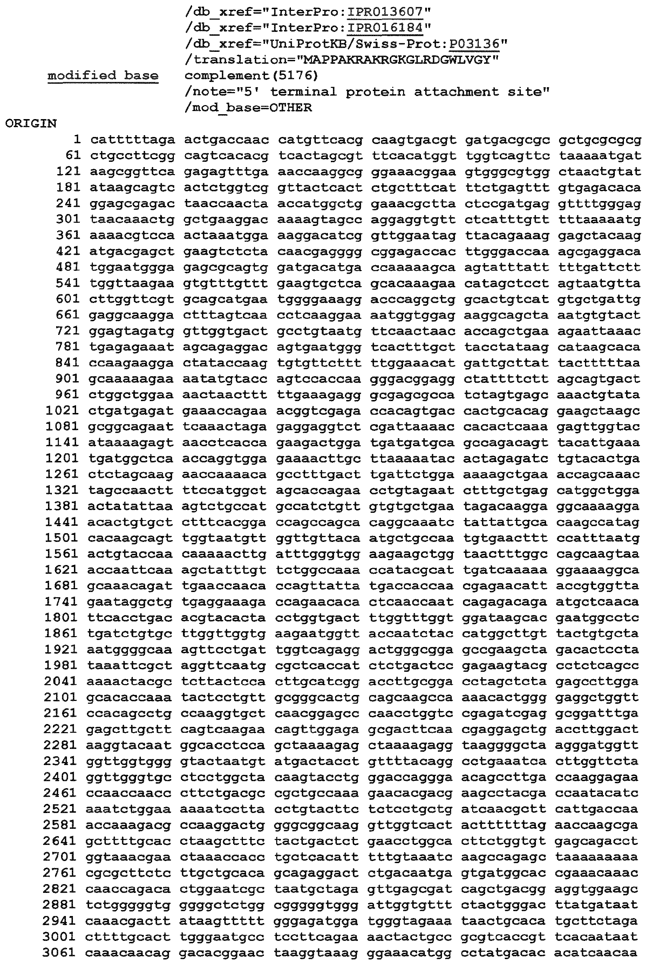

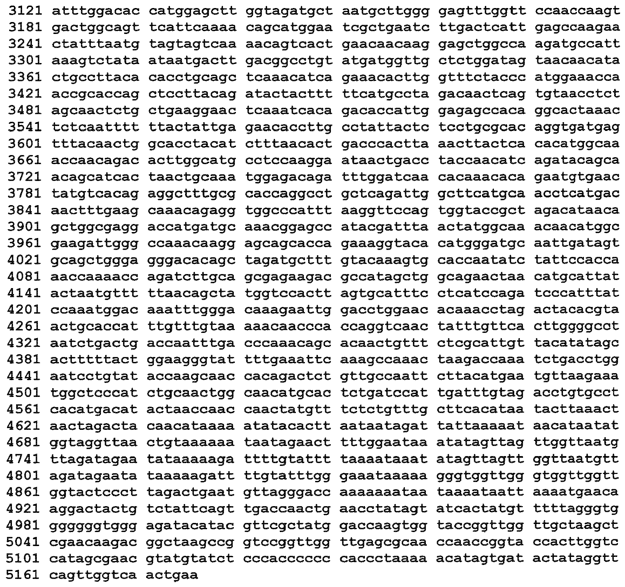

pΔH-1PVRNAi contains a ΔH-1PV viral genome featuring with an in-frame deletion of 114 nucleotide, from nucleotide 2022 to 2135 according to the NCBI gene bank (reference sequence X01457.1; Fig. 6) within the NS coding region [18]. The shRNA expression cassette at position nucleotide 4480 was introduced into pΔH-1PV by cloning a *Nsi*I*-Hpa*I fusion PCR DNA fragment generated with the primers indicated in Table 1 below. A particular preferred shPD-L1 in this study contains the sequence of 5'-GATATTTGCTGTCTTTATA-3 (SEQ ID NO:1) was possible to target all known isoforms of PD-L1 (GenBank Accession No. NM_014143). All plasmid constructs bearing shRNA expression cassette were further verified by sequencing (LGC Genomics, Berlin, Germany). The viruses were produced, purified, titrated by plaque assay and quantified by real-time PCR as previously described [26].

### Example 2

### Cell culture

The AsPC-1, U251 and HEK293T cell lines were grown in Dulbecco's modified Eagle's medium (DMEM, Sigma-Aldrich, Munich, Germany) supplemented with 10% fetal bovine serum (FBS, Gibco, Life Technologies, Darmstadt, Germany). PD-1/NFAT reporter Jurkat cells (BPS Bioscience, CA, USA) were cultured in Roswell Park Memorial Institute medium 1640 (RPMI, Invitrogen) supplemented with 10% FBS. NBK cells were grown in Minimum Essential Medium (MEM, Sigma-Aldrich, Munich, Germany), supplemented with 5% FBS. All media contained 2 mM 1-glutamine (Gibco), 100 U/ml penicillin and 100 µg/ml streptomycin (Gibco). All cells were grown at 37°C, 5% CO₂ atmosphere, 95% humidity and routinely checked for mycoplasma contamination using the Mycoplasma Detection Kit according to the manufacturer's instructions (Venor GeM, Minerva Biolabs, Berlin, Germany).

### Example 3

### Establishment of stable cell lines

Generation of AsPC-1 and U251 stable cell lines was carried out by using a pS/MARt DNA Vector expressing Flag tagged PD-L1 according the methods previously described [37]. Selection of positive clones was done on a medium containing 1µg/ml puromycin according to the manufacturer's protocol (Invitrogen, C10459). A pool of selected clones was used in this study.

### Example 4

### Evaluation of stability of the shRNA expression cassette.

Plasmids harboring the viral genome with shRNA expression cassette, were transiently transfected in HEK293T cells. After 3 days cells were harvested and viral particles released through three freeze- thaw cycles. Crude cell extracts were digested with Benzonase nuclease (Merck, Germany) and virus titers were quantified by real-time PCR and represented as encapsidated viral genomes per ml (Vg/ml) according to the methods previously described [26]. This period is defined as passage 0 stage (P0). The further amplification of the viral stocks was carried out in NBK producer cells using a fraction of the virus produced in HEK293T cells as inoculum. Cells were harvested after 3-5 days post-infection and treated as described above and this period is namely as passage 1 (P1). The viruses were harvested from every passage as illustrated in Fig. 2A. The virus DNA was extracted with Qiagen viral DNA extraction kit according to the manufacturer's protocol (Qiagen, Hilden, Germany). The genomic fragment including the shRNA cassette was amplified by PCR using primers flanking the cassette. The PCR product of ΔH-1PV was loaded as control.

### Example 5

### Protein extraction and Western blot analysis

Cells were scraped in the culture medium, harvested and washed with PBS. Cell pellets were lysed on ice for 30 min in lysis buffer (20 mM Tris-HCl at pH 7.5, 1 mM EDTA, 150 mM NaCl, 0.1% SDS, 1% Triton X-100, 1% Na-deoxycholate) containing protease (Roche Diagnostics, Mannheim, Germany) and phosphatase inhibitors (Sigma-Aldrich). Cell debris was removed by centrifugation at 13,000 rpm for 15 min at 4°C. The supernatants were kept at -80°C for further analysis. Total cell extracts (20 µg) were resolved by sodium dodecyl sulphate-polyacrylamide gel electrophoresis (SDS-PAGE) and transferred onto a Hybond-P membrane (GE Healthcare). The following antibodies were used for analysis: mouse monoclonal anti-vinculin (sc-25336; Santa Cruz Biotechnology, Heidelberg, Germany, used at 1:10,000 dilution), polyclonal anti-NS1 SP8 antiserum [31] (1:3,000), mouse monoclonal anti-Flag (AHP1074; AbD Serotec) at 1:2,000 dilution, rabbit anti-PD-Ll antibody at 1:1,000 dilution (PA5-20343, Thermo Fisher, USA). After incubation with horseradish peroxidase conjugated secondary antibodies (Santa Cruz Biotechnology) at 1:5,000 dilution, proteins were detected with ECL substrate solution in a Vilber Lourmat chemiluminescence detection system (Software, Chemi-Capt 5000).

### Example 6

### RNA isolation and real-time quantitative RT-PCR

Total RNA was isolated using the TRIzol^{®} Reagent RNA-extraction kit (Invitrogen) according to the manufacturer's instructions. 1 µg total cellular RNA was digested with 1 unit DNase I (Promega) at 37°C for 20 min to remove genomic DNA contamination, before processing for reverse transcription (RT) with oligo(dT) primers and reverse transcriptase of Moloney murine leukaemia virus (Promega). For each cDNA sample, a control was produced with an RT mixture to which no reverse transcriptase was added, in order to detect potential contamination of the cDNA sample with residual genomic DNA. Quantitative PCR using a fraction of the cDNA as a template was performed with the primer pairs in Table 1.

**Table 1. List of primers***

| **Primer name** | **Sequence** |
|---|---|
| Cloning | |
| Fusion PCR | F: 5'-GCAGAACTAACATGCATTATACTAATGTTTTT-3' |
| | R: 5'-ATTATTTTTTACAGTTAACCAATACCATATTA-3' |
| shRNAPD-Ll | F: 5'-TTAGATATTTGCTGTCTTTATACCTGACCCATATAAAGACAGCAAATATCTTTTTTGGAACTCTGTTTGCTTCACATAATAC-3' |
| | R: 5'-CAGAGTTCCAAAAAAGATATTTGCTGTCTTTATATGGGTCAGGTATAAAGACAGCAAATATCTAAGCTTAGATCTCT |
| shRNAEGFP | F: 5'-CTTAGCTGGAGTACAACTACAACCCTGACCCAGTTGTAGTTGTACTCCAGCTTTTTTGGAACTCTGTTTGCTTCACA-3' |
| | R: 5'-AAGCTGGAGTACAACTACAACTGGGTCAGGGTTGTAGTTGTACTCCAGCTAAGCTTAGATCTCTATCACTGATA-3' |
| RT-PCR | |
| PD-L1 | |
| transcript | F: 5'-TCAATGCCCCATACAACAAA-3' |
| | R: 5'-TGCTTGTCCAGATGACTTCG-3' |
| rRNA 18s | |
| transcript | F: 5'-CGCCTACCACATCCAAGGAA-3' |
| | R: 5'-GCTGGAATTACCGCGGCT-3' |

| | |
|---|---|
| *In the table, F indicates forward primer, R indicates reverse primer. | |

rRNA 18S was chosen as internal control as previously described [29]. The threshold cycle of fluorescence (Ct) for each sample was determined by real-time PCR using the Mastercycler^{®} ep realplex system (Eppendorf, Hamburg, Germany). Relative quantification of gene expression between the groups was performed applying the 2-ΔΔCt method [34]. Results are presented as fold expression compared to transcript levels of non-infected cells (mock).

### Example 7

### 1. Transfection and infection.

AsPC-1 and GBM U251 cells were seeded in 12-well plates at a density of 4 x 10⁵ and 2 x 10⁵/mL. After 24 h, cultures were transfected with 1 pg of vector expressing TCR activator (BPS Bioscience, CA, USA) as well as plasmids expressing TCR activator/PD-L1 (BPS Bioscience, CA, USA) in the presence of 3 µl metafectene reagent according to manufacturer's instructions (Biontex Laboratories GmbH, Munich, Germany). After 24 h, cells were trypsinized and split in 96-well plates at a density of 4,2 x 10⁴ for AsPC-1 and 2 x 10⁴ for GBM U251 cell line, meanwhile keeping the transfected cell lines at number with 4 x 10⁵ of cells in 6 cm dish for evaluation of PD-L1 gene expression upon treatments. The cells either in 96-well plates or 6 cm dished were incubated for 8 h after splitting and then infected (or not) with either ΔH-1PVshPD-L1 or ΔH-1PVshEGFP at a MOI (pfu/cell) of 12 (AsPC-1) and 2 (U251).

### 2. Co-culture with PD-1/NFAT reporter Jurkat cells.

PD-1/NFAT reporter Jurkat cells were purchased from BPS Bioscience (CA, USA). Briefly, the infected cells in 96-well plates at post-infection 68 h, were pre-incubated with either anti-PD-Ll neutralizing (#71213, BPS Bioscience, CA, USA) or control antibodies (PA5-20343, Thermo Fisher, USA) at dilution 50 ng/ml in the medium according to manufacturer's instructions and incubate for 30 min at 37°C followed by co-culture with 6 x 10⁴ Jurkat cells per well for 16 h at 37 °C incubator. After incubation, the ONE-Step luciferase assay (BPS Bioscience, CA, USA) was performed by adding the luciferase reagent to treated wells as well as untreated controls for 30 min followed by luminescence measurement. The induction of NFAT luciferase reporter expression under either different treatments or controls, is calculated as average of background-subtracted luminescence. Results are presented as a relative ratio of average luminescence of TCR activator/PD-L1 expressed sample versus average luminescence of TCR activator alone expressed sample under different treatments as well as controls.

### List of References

1 Fire, A., Xu, S., Montgomery, M. K., Kostas, S. A., Driver, S. E. and Mello, C. C. (1998) Potent and specific genetic interference by double-stranded RNA in Caenorhabditis elegans. Nature. 391, 806-811
2 Davidson, B. L. and McCray, P. B., Jr. (2011) Current prospects for RNA interference-based therapies. Nat Rev Genet. 12, 329-340
3 Dorsett, Y. and Tuschl, T. (2004) siRNAs: applications in functional genomics and potential as therapeutics. Nat Rev Drug Discov. 3, 318-329
4 Rettig, G. R. and Behlke, M. A. (2012) Progress toward in vivo use of siRNAs-II. Molecular therapy : the journal of the American Society of Gene Therapy. 20, 483-512
5 Bobbin, M. L. and Rossi, J. J. (2016) RNA Interference (RNAi)-Based Therapeutics: Delivering on the Promise? Annual review of pharmacology and toxicology. 56, 103-122
6 Snove, O., Jr. and Rossi, J. J. (2006) Expressing short hairpin RNAs in vivo. Nat Methods. 3, 689-695
7 Brummelkamp, T. R., Bernards, R. and Agami, R. (2002) A system for stable expression of short interfering RNAs in mammalian cells. Science. 296, 550-553
8 Yoo, J. Y., Kim, J. H., Kwon, Y. G., Kim, E. C., Kim, N. K., Choi, H. J. and Yun, C. O. (2007) VEGF-specific short hairpin RNA-expressing oncolytic adenovirus elicits potent inhibition of angiogenesis and tumor growth. Mol Ther. 15, 295-302
9 Mao, L. J., Zhang, J., Liu, N., Fan, L., Yang, D. R., Xue, B. X., Shan, Y. X. and Zheng, J. N. (2015) Oncolytic virus carrying shRNA targeting SATB1 inhibits prostate cancer growth and metastasis. Tumour biology : the journal of the International Society for Oncodevelopmental Biology and Medicine. 36, 9073-9081
10 Jung, S. H., Choi, J. W., Yun, C. O., Yhee, J. Y., Price, R., Kim, S. H., Kwon, I. C. and Ghandehari, H. (2014) Sustained local delivery of oncolytic short hairpin RNA adenoviruses for treatment of head and neck cancer. J Gene Med. 16, 143-152
11 Fang, L., Cheng, Q., Li, W., Liu, J., Li, L., Xu, K. and Zheng, J. (2014) Antitumor activities of an oncolytic adenovirus equipped with a double siRNA targeting Ki67 and hTERT in renal cancer cells. Virus Res. 181, 61-71
12 Li, Y., Zhang, B., Zhang, H., Zhu, X., Feng, D., Zhang, D., Zhuo, B., Li, L. and Zheng, J. (2013) Oncolytic adenovirus armed with shRNA targeting MYCN gene inhibits neuroblastoma cell proliferation and in vivo xenograft tumor growth. Journal of cancer research and clinical oncology. 139, 933-941
13 Kim, J., Nam, H. Y., Kim, T. I., Kim, P. H., Ryu, J., Yun, C. O. and Kim, S. W. (2011) Active targeting of RGD-conjugated bioreducible polymer for delivery of oncolytic adenovirus expressing shRNA against IL-8 mRNA. Biomaterials. 32, 5158-5166
14 Gao, Y., Zhu, Y., Huang, X., Ai, K., Zheng, Q. and Yuan, Z. (2015) Gene therapy targeting hepatocellular carcinoma by a dual-regulated oncolytic adenovirus harboring the focal adhesion kinase shRNA. International journal of oncology. 47, 668-678
15 Zheng, J. N., Pei, D. S., Mao, L. J., Liu, X. Y., Mei, D. D., Zhang, B. F., Shi, Z., Wen, R. M. and Sun, X. Q. (2009) Inhibition of renal cancer cell growth in vitro and in vivo with oncolytic adenovirus armed short hairpin RNA targeting Ki-67 encoding mRNA. Cancer Gene Ther. 16, 20-32
16 Anesti, A. M., Simpson, G. R., Price, T., Pandha, H. S. and Coffin, R. S. (2010) Expression of RNA interference triggers from an oncolytic herpes simplex virus results in specific silencing in tumour cells in vitro and tumours in vivo. BMC cancer. 10, 486
17 Marchini, A., Bonifati, S., Scott, E. M., Angelova, A. L. and Rommelaere, J. (2015) Oncolytic parvoviruses: from basic virology to clinical applications. Virol J. 12, 6
18 Weiss, N., Stroh-Dege, A., Rommelaere, J., Dinsart, C. and Salome, N. (2012) An in-frame deletion in the NS protein-coding sequence of parvovirus H-1PV efficiently stimulates export and infectivity of progeny virions. J Virol. 86, 7554-7564
19 Illarionova, A., Rommelaere, J., Leuchs, B. and Marchini, A. (2012) Modified parvovirus useful for gene silencing. EP2620503
20 Morales, F. and Giordano, A. (2016) Overview of CDK9 as a target in cancer research. Cell Cycle. 15, 519-527
21 Wang, S. and Fischer, P. M. (2008) Cyclin-dependent kinase 9: a key transcriptional regulator and potential drug target in oncology, virology and cardiology. Trends Pharmacol Sci. 29, 302-313
22 Sims, R. J., 3rd, Belotserkovskaya, R. and Reinberg, D. (2004) Elongation by RNA polymerase II: the short and long of it. Genes Dev. 18, 2437-2468
23 Shapiro, G. I. (2006) Cyclin-dependent kinase pathways as targets for cancer treatment. J Clin Oncol. 24, 1770-1783
24 Romano, G. and Giordano, A. (2008) Role of the cyclin-dependent kinase 9-related pathway in mammalian gene expression and human diseases. Cell Cycle. 7, 3664-3668
25 Krystof, V., Baumli, S. and Furst, R. (2012) Perspective of cyclin-dependent kinase 9 (CDK9) as a drug target. Curr Pharm Des. 18, 2883-2890
26 Leuchs, B., Roscher, M., Muller, M., Kurschner, K. and Rommelaere, J. (2016) Standardized large-scale H-1PV production process with efficient quality and quantity monitoring. J Virol Methods. 229, 48-59
27 Laemmli, U. K. (1970) Cleavage of structural proteins during the assembly of the head of bacteriophage T4. Nature. 227, 680-685
28 Nicoletti, I., Migliorati, G., Pagliacci, M. C., Grignani, F. and Riccardi, C. (1991) A rapid and simple method for measuring thymocyte apoptosis by propidium iodide staining and flow cytometry. J Immunol Methods. 139, 271-279
29 Li, J., Bonifati, S., Hristov, G., Marttila, T., Valmary-Degano, S., Stanzel, S., Schnolzer, M., Mougin, C., Aprahamian, M., Grekova, S. P., Raykov, Z., Rommelaere, J. and Marchini, A. (2013) Synergistic combination of valproic acid and oncolytic parvovirus H-1PV as a potential therapy against cervical and pancreatic carcinomas. EMBO Mol Med. 5, 1537-1555
30 Kestler, J., Neeb, B., Struyf, S., Van Damme, J., Cotmore, S. F., D'Abramo, A., Tattersall, P., Rommelaere, J., Dinsart, C. and Cornelis, J. J. (1999) cis requirements for the efficient production of recombinant DNA vectors based on autonomous parvoviruses. Hum Gene Ther. 10, 1619-1632
31 Bodendorf, U., Cziepluch, C., Jauniaux, JC., Rommelaere, J., Salome, N. (1999) Nuclear export factor CRM1 interacts with nonstructural proteins NS2 from parvovirus minute virus of mice. J Virol 73, 7769-7779.
32 Breton G, Yassine-Diab B, Cohn L, Boulassel MR, Routy JP, Sekaly RP, Steinman RM. (2009) siRNA knockdown of PD-L1 and PD-L2 in monocyte-derived dendritic cells only modestly improves proliferative responses to Gag by CD8(+) T cells from HIV-1-infected individuals. J. Clin. Immunol. 29, 637-645.
33 Jeffery JM, Urquhart AJ, Subramaniam VN, Parton RG, Khanna KK. (2010) Centrobin regulates the assembly of functional mitotic spindles. Oncogene. 29, 2649-2658.
34 Livak, K.J, Schmittgen, TD. (2001) Analysis of relative gene expression data using real-time quantitative PCR and the 2(-Delta Delta C(T)) Method. Methods. 25, 402-408.
35 Sun C, Mezzadra R, Schumacher TN. (2018). Regulation and function of the PD-L1 checkpoint. Immunity. 48, 434-452.
36 Wang S, Wang Y, Liu J, Shao S, Li X, Gao J, Niu H, Wang X. (2014) Silencing B7-H1 enhances the anti-tumor effect of bladder cancer antigen-loaded dendritic cell vaccine in vitro. Onco Targets Ther. 7,1389-1396.
37 Sagini MN, Zepp M, Bergmann F, Bozza M, Harbottle R, Berger MR. (2018) The expression of genes contributing to pancreatic adenocarcinoma progression is influenced by the respective environment. Genes Cancer. 9,114-129.

## Claims

1. Parvovirus for down regulating the expression of a target specific nucleic acid in a cell **characterized in that** it is derived from a parvovirus H-1 deletion variant containing a deletion encompassing the nucleotides 2022-2135, wherein a target specific nucleic acid is inserted at nucleotide 4480 of the H-1 parvovirus VP gene and is expressible under the control of a promoter or promoter region recognizable by an RNA polymerase in the cell, wherein said target specific nucleic acid is transcribable in an RNAi, and wherein said parvovirus is capable of replicating and propagating in the cell.

2. The parvovirus of claim 1, wherein the target specific nucleic acid is a PD-L1 nucleic acid.

3. The parvovirus of claim 1 or 2, wherein the promoter or promoter region recognizable by a RNA polymerase of the cell is an RNA-polymerase III (Pol III) promoter.

4. The parvovirus of claim 3, wherein the RNA-polymerase III (Pol III) promoter is the RNA-polymerase III H1 promoter.

5. The parvovirus of any one of claims 1 to 4, wherein the target specific nucleic acid is an shRNA.

6. The parvovirus of any one of claims 1 to 5, wherein the target specific nucleic acid has a length of at least 15 nucleotides.

7. The parvovirus of claim 5, wherein the target specific nucleic acid matches the sequence 5'GATATTTGCTGTCTTTATA-3' (SEQ ID NO:1) of the PD-L1 sequence.

8. The parvovirus according to any one of claims 1 to 7 for the use in a method of treating a tumour.

9. The parvovirus for the use according to claim 8, **characterized in that** the cells of said tumour are resistant to chemotherapy and/or radiotherapy.

10. The parvovirus for the use according to claim 8 or 9, **characterized in that** said parvovirus is administered by intravenous (i.v.), intratumoral or endobronchial administration.

11. An isolated cell containing a parvovirus of any one of claims 1 to 7.

12. A transgenic non-human animal comprising a cell of claim 11.


SEQUENCE LISTING

<110> Deutsches Krebsforschungszentrum

<120> H-1 PV expressing RNAi effectors

<130> K 3668EP

<160> 13

<170> BiSSAP 1.3.6

<210> 1
<211> 19
<212> DNA
<213> Homo sapiens

<400> 1
gatatttgct gtctttata                                                    19

<210> 2
<211> 32
<212> DNA
<213> Artificial Sequence

<220>
<223> primer forward

<400> 2
gcagaactaa catgcattat actaatgttt tt                                     32

<210> 3
<211> 32
<212> DNA
<213> Artificial Sequence

<220>
<223> primer reverse

<400> 3
attatttttt acagttaacc aataccatat ta                                     32

<210> 4
<211> 82
<212> DNA
<213> Artificial Sequence

<220>
<223> primer forward

<400> 4
   ttagatattt gctgtcttta tacctgaccc atataaagac agcaaatatc ttttttggaa      60
   ctctgtttgc ttcacataat ac                                                82

<210> 5
<211> 77
<212> DNA
<213> Artificial Sequence

<220>
<223> primer reverse

<400> 5
cagagttcca aaaaagatat ttgctgtctt tatatgggtc aggtataaag acagcaaata 60

tctaagctta gatctct 77

<210> 6
<211> 77
<212> DNA
<213> Artificial Sequence

<220>
<223> primer forward

<400> 6
cttagctgga gtacaactac aaccctgacc cagttgtagt tgtactccag cttttttgga 60

actctgtttg cttcaca 77

<210> 7
<211> 74
<212> DNA
<213> Artificial Sequence

<220>
<223> primer reverse

<400> 7
aagctggagt acaactacaa ctgggtcagg gttgtagttg tactccagct aagcttagat 60

ctctatcact gata 74

<210> 8
<211> 20
<212> DNA
<213> Artificial Sequence

<220>
<223> primer forward

<400> 8
tcaatgcccc atacaacaaa 20

<210> 9
<211> 20
<212> DNA
<213> Artificial Sequence

<220>
<223> primer reverse

<400> 9
tgcttgtcca gatgacttcg                                                   20


<210> 10
<211> 20
<212> DNA
<213> Artificial Sequence


<220>
<223> primer forward

<400> 10
cgcctaccac atccaaggaa                                                   20


<210> 11
<211> 18
<212> DNA
<213> Artificial Sequence


<220>
<223> primer reverse

<400> 11
gctggaatta ccgcggct                                                     18


<210> 12
<211> 672
<212> PRT
<213> Parvovirus


<400> 12
Met Ala Gly Asn Ala Tyr Ser Asp Glu Val Leu Gly Val Thr Asn Trp
1               5                   10                  15
Leu Lys Asp Lys Ser Ser Gln Glu Val Phe Ser Phe Val Phe Lys Asn
            20                  25                  30
Glu Asn Val Gln Leu Asn Gly Lys Asp Ile Gly Trp Asn Ser Tyr Arg
        35                  40                  45
Lys Glu Leu Gln Asp Asp Glu Leu Lys Ser Leu Gln Arg Gly Ala Glu
    50                  55                  60
Thr Thr Trp Asp Gln Ser Glu Asp Met Glu Trp Glu Ser Ala Val Asp
65                  70                  75                  80
Asp Met Thr Lys Lys Gln Val Phe Ile Phe Asp Ser Leu Val Lys Lys
                85                  90                  95
Cys Leu Phe Glu Val Leu Ser Thr Lys Asn Ile Ala Pro Ser Asn Val
            100                 105                 110
Thr Trp Phe Val Gln His Glu Trp Gly Lys Asp Pro Gly Trp His Cys
        115                 120                 125
His Val Leu Ile Gly Gly Lys Asp Phe Ser Gln Pro Gln Gly Lys Trp
    130                 135                 140
Trp Arg Arg Gln Leu Asn Val Tyr Trp Ser Arg Trp Leu Val Thr Ala
145                 150                 155                 160
Cys Asn Val Gln Leu Thr Pro Ala Glu Arg Ile Lys Leu Arg Glu Ile
                165                 170                 175

Ala Glu Asp Ser Glu Trp Val Thr Leu Leu Thr Tyr Lys His Lys His
            180                 185                 190
Thr Lys Lys Asp Tyr Thr Lys Cys Val Leu Phe Gly Asn Met Ile Ala
        195                 200                 205
Tyr Tyr Phe Leu Ser Lys Lys Lys Ile Cys Thr Ser Pro Pro Arg Asp
    210                 215                 220
Gly Gly Tyr Phe Leu Ser Ser Asp Ser Gly Trp Lys Thr Asn Phe Leu
225                 230                 235                 240
Lys Glu Gly Glu Arg His Leu Val Ser Lys Leu Tyr Thr Asp Glu Met
                245                 250                 255
Lys Pro Glu Thr Val Glu Thr Thr Val Thr Thr Ala Gln Glu Ala Lys
            260                 265                 270
Arg Gly Arg Ile Gln Thr Arg Glu Glu Val Ser Ile Lys Thr Thr Leu
        275                 280                 285
Lys Glu Leu Val His Lys Arg Val Thr Ser Pro Glu Asp Trp Met Met
    290                 295                 300
Met Gln Pro Asp Ser Tyr Ile Glu Met Met Ala Gln Pro Gly Gly Glu
305                 310                 315                 320
Asn Leu Leu Lys Asn Thr Leu Glu Ile Cys Thr Leu Thr Leu Ala Arg
                325                 330                 335
Thr Lys Thr Ala Phe Asp Leu Ile Leu Glu Lys Ala Glu Thr Ser Lys
            340                 345                 350
Leu Ala Asn Phe Ser Met Ala Ser Thr Arg Thr Cys Arg Ile Phe Ala
        355                 360                 365
Glu His Gly Trp Asn Tyr Ile Lys Val Cys His Ala Ile Cys Cys Val
    370                 375                 380
Leu Asn Arg Gln Gly Gly Lys Arg Asn Thr Val Leu Phe His Gly Pro
385                 390                 395                 400
Ala Ser Thr Gly Lys Ser Ile Ile Ala Gln Ala Ile Ala Gln Ala Val
                405                 410                 415
Gly Asn Val Gly Cys Tyr Asn Ala Ala Asn Val Asn Phe Pro Phe Asn
            420                 425                 430
Asp Cys Thr Asn Lys Asn Leu Ile Trp Val Glu Glu Ala Gly Asn Phe
        435                 440                 445
Gly Gln Gln Val Asn Gln Phe Lys Ala Ile Cys Ser Gly Gln Thr Ile
    450                 455                 460
Arg Ile Asp Gln Lys Gly Lys Gly Ser Lys Gln Ile Glu Pro Thr Pro
465                 470                 475                 480
Val Ile Met Thr Thr Asn Glu Asn Ile Thr Val Val Arg Ile Gly Cys
                485                 490                 495
Glu Glu Arg Pro Glu His Thr Gln Pro Ile Arg Asp Arg Met Leu Asn
            500                 505                 510
Ile His Leu Thr Arg Thr Leu Pro Gly Asp Phe Gly Leu Val Asp Lys
        515                 520                 525
His Glu Trp Pro Leu Ile Cys Ala Trp Leu Val Lys Asn Gly Tyr Gln
    530                 535                 540
Ser Thr Met Ala Cys Tyr Cys Ala Lys Trp Gly Lys Val Pro Asp Trp
545                 550                 555                 560
Ser Glu Asp Trp Ala Glu Pro Lys Leu Asp Thr Pro Ile Asn Ser Leu
                565                 570                 575
Gly Ser Met Arg Ser Pro Ser Leu Thr Pro Arg Ser Thr Pro Leu Ser
            580                 585                 590
Gln Asn Tyr Ala Leu Thr Pro Leu Ala Ser Asp Leu Ala Asp Leu Ala
        595                 600                 605
Leu Glu Pro Trp Ser Thr Pro Asn Thr Pro Val Ala Gly Thr Ala Ala
    610                 615                 620
Ser Gln Asn Thr Gly Glu Ala Gly Ser Thr Ala Cys Gln Gly Ala Gln
625                 630                 635                 640
Arg Ser Pro Thr Trp Ser Glu Ile Glu Ala Asp Leu Arg Ala Cys Phe
                645                 650                 655
Ser Gln Glu Gln Leu Glu Ser Asp Phe Asn Glu Glu Leu Thr Leu Asp
            660                 665                 670

<210> 13
<211> 5176
<212> DNA
<213> Parvovirus

<400> 13
catttttaga actgaccaac catgttcacg caagtgacgt gatgacgcgc gctgcgcgcg 60

ctgccttcgg cagtcacacg tcactagcgt ttcacatggt tggtcagttc taaaaatgat 120

aagcggttca gagagtttga aaccaaggcg ggaaacggaa gtgggcgtgg ctaactgtat 180

ataagcagtc actctggtcg gttactcact ctgctttcat ttctgagttt gtgagacaca 240

ggagcgagac taaccaacta accatggctg gaaacgctta ctccgatgag gttttgggag 300

taacaaactg gctgaaggac aaaagtagcc aggaggtgtt ctcatttgtt tttaaaaatg 360

aaaacgtcca actaaatgga aaggacatcg gttggaatag ttacagaaag gagctacaag 420

atgacgagct gaagtctcta caacgagggg cggagaccac ttgggaccaa agcgaggaca 480

tggaatggga gagcgcagtg gatgacatga ccaaaaagca agtatttatt tttgattctt 540

tggttaagaa gtgtttgttt gaagtgctca gcacaaagaa catagctcct agtaatgtta 600

cttggttcgt gcagcatgaa tggggaaagg acccaggctg gcactgtcat gtgctgattg 660

gaggcaagga ctttagtcaa cctcaaggaa aatggtggag aaggcagcta aatgtgtact 720

ggagtagatg gttggtgact gcctgtaatg ttcaactaac accagctgaa agaattaaac 780

tgagagaaat agcagaggac agtgaatggg tcactttgct tacctataag cataagcaca 840

ccaagaagga ctataccaag tgtgttcttt ttggaaacat gattgcttat tactttttaa 900

gcaaaaagaa aatatgtacc agtccaccaa gggacggagg ctattttctt agcagtgact 960

ctggctggaa aactaacttt ttgaaagagg gcgagcgcca tctagtgagc aaactgtata 1020

ctgatgagat gaaaccagaa acggtcgaga ccacagtgac cactgcacag gaagctaagc 1080

gcggcagaat tcaaactaga gaggaggtct cgattaaaac cacactcaaa gagttggtac 1140

ataaaagagt aacctcacca gaagactgga tgatgatgca gccagacagt tacattgaaa 1200

tgatggctca accaggtgga gaaaacttgc ttaaaaatac actagagatc tgtacactga 1260

ctctagcaag aaccaaaaca gcctttgact tgattctgga aaaagctgaa accagcaaac 1320

tagccaactt ttccatggct agcaccagaa cctgtagaat ctttgctgag catggctgga 1380

actatattaa agtctgccat gccatctgtt gtgtgctgaa tagacaagga ggcaaaagga 1440

acactgtgct ctttcacgga ccagccagca caggcaaatc tattattgca caagccatag 1500

cacaagcagt tggtaatgtt ggttgttaca atgctgccaa tgtgaacttt ccatttaatg 1560

actgtaccaa caaaaacttg atttgggtgg aagaagctgg taactttggc cagcaagtaa 1620

accaattcaa agctatttgt tctggccaaa ccatacgcat tgatcaaaaa ggaaaaggca 1680

gcaaacagat tgaaccaaca ccagttatta tgaccaccaa cgagaacatt accgtggtta 1740

gaataggctg tgaggaaaga ccagaacaca ctcaaccaat cagagacaga atgctcaaca 1800

ttcacctgac acgtacacta cctggtgact ttggtttggt ggataagcac gaatggcctc 1860

tgatctgtgc ttggttggtg aagaatggtt accaatctac catggcttgt tactgtgcta 1920

aatggggcaa agttcctgat tggtcagagg actgggcgga gccgaagcta gacactccta 1980

taaattcgct aggttcaatg cgctcaccat ctctgactcc gagaagtacg cctctcagcc 2040

aaaactacgc tcttactcca cttgcatcgg accttgcgga cctagctcta gagccttgga 2100

gcacaccaaa tactcctgtt gcgggcactg cagcaagcca aaacactggg gaggctggtt 2160

ccacagcctg ccaaggtgct caacggagcc caacctggtc cgagatcgag gcggatttga 2220

gagcttgctt cagtcaagaa cagttggaga gcgacttcaa cgaggagctg accttggact 2280

aaggtacaat ggcacctcca gctaaaagag ctaaaagagg taaggggcta agggatggtt 2340

ggttggtggg gtactaatgt atgactacct gttttacagg cctgaaatca cttggttcta 2400

ggttgggtgc ctcctggcta caagtacctg ggaccaggga acagccttga ccaaggagaa 2460

ccaaccaacc cttctgacgc cgctgccaaa gaacacgacg aagcctacga ccaatacatc 2520

aaatctggaa aaaatcctta cctgtacttc tctcctgctg atcaacgctt cattgaccaa 2580

accaaagacg ccaaggactg gggcggcaag gttggtcact acttttttag aaccaagcga 2640

gcttttgcac ctaagctttc tactgactct gaacctggca cttctggtgt gagcagacct 2700

ggtaaacgaa ctaaaccacc tgctcacatt tttgtaaatc aagccagagc taaaaaaaaa 2760

cgcgcttctc ttgctgcaca gcagaggact ctgacaatga gtgatggcac cgaaacaaac 2820

caaccagaca ctggaatcgc taatgctaga gttgagcgat cagctgacgg aggtggaagc 2880

tctgggggtg ggggctctgg cgggggtggg attggtgttt ctactgggac ttatgataat 2940

caaacgactt ataagttttt gggagatgga tgggtagaaa taactgcaca tgcttctaga 3000

cttttgcact tgggaatgcc tccttcagaa aactactgcc gcgtcaccgt tcacaataat 3060

caaacaacag gacacggaac taaggtaaag ggaaacatgg cctatgacac acatcaacaa 3120

atttggacac catggagctt ggtagatgct aatgcttggg gagtttggtt ccaaccaagt 3180

gactggcagt tcattcaaaa cagcatggaa tcgctgaatc ttgactcatt gagccaagaa 3240

ctatttaatg tagtagtcaa aacagtcact gaacaacaag gagctggcca agatgccatt 3300

aaagtctata ataatgactt gacggcctgt atgatggttg ctctggatag taacaacata 3360

ctgccttaca cacctgcagc tcaaacatca gaaacacttg gtttctaccc atggaaacca 3420

accgcaccag ctccttacag atactacttt ttcatgccta gacaactcag tgtaacctct 3480

agcaactctg ctgaaggaac tcaaatcaca gacaccattg gagagccaca ggcactaaac 3540

tctcaatttt ttactattga gaacaccttg cctattactc tcctgcgcac aggtgatgag 3600

tttacaactg gcacctacat ctttaacact gacccactta aacttactca cacatggcaa 3660

accaacagac acttggcatg cctccaagga ataactgacc taccaacatc agatacagca 3720

acagcatcac taactgcaaa tggagacaga tttggatcaa cacaaacaca gaatgtgaac 3780

tatgtcacag aggctttgcg caccaggcct gctcagattg gcttcatgca acctcatgac 3840

aactttgaag caaacagagg tggcccattt aaggttccag tggtaccgct agacataaca 3900

gctggcgagg accatgatgc aaacggagcc atacgattta actatggcaa acaacatggc 3960

gaagattggg ccaaacaagg agcagcacca gaaaggtaca catgggatgc aattgatagt 4020

gcagctggga gggacacagc tagatgcttt gtacaaagtg caccaatatc tattccacca 4080

aaccaaaacc agatcttgca gcgagaagac gccatagctg gcagaactaa catgcattat 4140

actaatgttt ttaacagcta tggtccactt agtgcatttc ctcatccaga tcccatttat 4200

ccaaatggac aaatttggga caaagaattg gacctggaac acaaacctag actacacgta 4260

actgcaccat ttgtttgtaa aaacaaccca ccaggtcaac tatttgttca cttggggcct 4320

aatctgactg accaatttga cccaaacagc acaactgttt ctcgcattgt tacatatagc 4380

actttttact ggaagggtat tttgaaattc aaagccaaac taagaccaaa tctgacctgg 4440

aatcctgtat accaagcaac cacagactct gttgccaatt cttacatgaa tgttaagaaa 4500

tggctcccat ctgcaactgg caacatgcac tctgatccat tgatttgtag acctgtgcct 4560

cacatgacat actaaccaac caactatgtt tctctgtttg cttcacataa tacttaaact 4620

aactagacta caacataaaa atatacactt aataatagat tattaaaaat aacataatat 4680

ggtaggttaa ctgtaaaaaa taatagaact tttggaataa atatagttag ttggttaatg 4740

ttagatagaa tataaaaaga ttttgtattt taaaataaat atagttagtt ggttaatgtt 4800

agatagaata taaaaagatt ttgtatttgg gaaataaaaa gggtggttgg gtggttggtt 4860

ggtactccct tagactgaat gttaggacca aaaaaaataa taaaataatt aaaatgaaca 4920

aggactactg tctattcagt tgaccaactg aacctatagt atcactatgt ttttagggtg 4980

ggggggtggg agatacatac gttcgctatg gaccaagtgg taccggttgg ttgctaagct 5040

cgaacaagac ggctaagccg gtccggttgg ttgagcgcaa ccaaccggta ccacttggtc 5100

catagcgaac gtatgtatct cccacccccc caccctaaaa acatagtgat actataggtt 5160

cagttggtca actgaa 5176